# EUROPÄISCHE PATENTANMELDUNG

(11) **EP 3 756 556 A1**
(43) Veröffentlichungstag der Anmeldung: **30.12.2020**
(21) Anmeldenummer: 19183164.3
(22) Anmeldetag: 28.06.2019
(51) Int. Cl.: A61B 17/064, A61B 17/10, A61B 50/30, A61B 17/00, A61B 90/00

(54) **CHIRURGISCHES WERKZEUG**

(71) Anmelder: Medartis Holding AG, 4057 Basel (CH)
(72) Erfinder: Langer, Barry, 4106 Therwil (CH)
(74) Vertreter: Hepp Wenger Ryffel AG

(57) **Zusammenfassung**

Offenbart sind chirurgische Werkezeuge (10), die eine chirurgische Klammer (20) mit mindestens zwei, bevorzugt genau zwei Schenkeln (21) und einer die Schenkel (21) verbindenden Basis (22) enthalten. Die Werkzeuge (10) weisen eines oder mehrere der folgenden weiteren Elemente auf: einen mit der Klammer (20) einstückig verbundenen Haltegriff (30), wobei der Haltegriff (30) über mindestens eine erste Sollbruchstelle (91) mit der Basis (22) der Klammer (20) verbunden ist; eine Bohrhilfe (40) mit mindestens zwei Öffnungen (41), deren Abstand (D) grösser ist als der Abstand (d) der Schenkel (21) der Klammer (20); einen Bohrer (50), der, insbesondere an einem ersten Ende (51), über mindestens eine dritte Sollbruchstelle (93) mit dem Haltegriff (30) verbunden ist; eine Abtrennöffnung (42) welche derart ausgebildet ist, dass, insbesondere nach einer Abtrennung der Bohrhilfe (40) vom Haltegriff (30), die Klammer insbesondere im Bereich der ersten Sollbruchstelle (91) derart in der Abtrennöffnung (42) aufnehmbar ist, dass die Klammer (20) an der ersten Sollbruchstelle (91) vom Haltegriff (30) abtrennbar ist. Allgemeiner sind auch einstückige chirurgische Werkzeuge (10; 10') offenbart, die mindestens zwei Elemente aufweisen, die durch Sollbruchstellen miteinander verbunden sind.

## Beschreibung

Die vorliegende Erfindung betrifft chirurgische Werkzeuge gemäss dem Oberbegriff der unabhängigen Patentansprüche. Es sind Werkzeuge bekannt, die mit Klammern versehen sind, die Einsatz in der Knochenchirurgie finden können, insbesondere zur Kompression von Knochen oder deren Fragmenten.

EP 2 106 754 A1 offenbart chirurgische Instrumente mit einer Klammer und zwei Haltegriffen, die über Sollbruchstellen mit der Klammer verbunden sind. In den Haltegriffen sind Öffnungen zur Aufnahme eines Werkzeugs vorgesehen. Damit kann die Klammer auf- oder zugebogen werden.

WO 2004/004578 A1 offenbart Werkzeuge mit einer Klammer und einem Halteteil, das über geschwächte Laschen mit der Klammer verbunden ist.

Die in EP 2 474 271 A2 offenbarten Instrumente verfügen über Halteteile, die über vertiefte Sollbruchstellen mit einer Klammer verbunden sind. Die Sollbruchstellen liegen in einem Eckbereich zwischen Schenkeln und Basis der Klammer. Im Halteteil sind Schablonenbohrungen und Ausnehmungen vorgesehen.

Weiterhin offenbart DE 198 59 950 A1 eine Klammer, die innerhalb einer Ausnehmung eines Werkstoffblattes angeordnet ist.

Die aus diesem Stand der Technik bekannten chirurgischen Werkzeuge weisen allerdings eine Reihe von Nachteilen auf. So können beispielsweise beim Abtrennen der in EP 2 474 271 A2 offenbarten Klammern scharfkantige Konturen entstehen, die dem Körpergewebe beim Einsetzen der Klammer Schaden zufügen können. Des Weiteren sind bei der Verwendung der Klammern vielfach weitere Werkzeuge zum Bohren von Löchern, zum Abtrennen und zum Einsetzen erforderlich, die auf die Klammern individuell abgestimmt sein müssen, was die Bereitstellung erschwert.

Es ist daher eine Aufgabe der vorliegenden Erfindung, chirurgische Werkzeuge mit chirurgischen Klammern bereit zu stellen, bei denen die genannten Nachteile ausgeräumt oder zumindest reduziert sind. Insbesondere sollen die Klammern das Körpergewebe möglichst wenig beschädigen, und die Bereitstellung weiterer, für die Benutzung der Klammern erforderlicher Werkzeuge soll möglichst vereinfacht werden.

In einem ersten Aspekt betrifft die Erfindung ein chirurgisches Werkzeug mit einer chirurgischen Klammer und einem Haltegriff. Die Klammer weist mindestens zwei, bevorzugt genau zwei Schenkel und eine die Schenkel verbindende Basis auf. Der Haltegriff ist mit der Klammer einstückig verbunden. Gemäss dem ersten Aspekt der Erfindung ist der Haltegriff über mindestens eine erste Sollbruchstelle mit der Basis der Klammer verbunden. Aufgrund der Anordnung der ersten Sollbruchstelle an der Basis der Klammer entstehen beim Abtrennen keine scharfen Kanten an den Schenkeln der Klammer. Hierdurch wird das Körpergewebe postoperativ geschont.

Unter einer Sollbruchstelle wird hier und im Folgenden ein Stelle des Werkzeugs verstanden, die einen ersten Teil des Werkzeugs (beispielsweise den Haltegriff) mit einem zweiten Teil des Werkzeugs (beispielsweise mit der Klammer) verbindet, wobei diese Verbindung durch Aufbringung eines Bruchdrehmoments gezielt an dieser Stelle getrennt werden kann. Dabei kann das Bruchdrehmoment im Bereich von 0,1 Nm bis 2 Nm liegen. Die Sollbruchstelle kann beispielsweise durch eine Stelle des Werkzeugs gebildet werden, die dünner ist als benachbarte Bereiche des Werkzeugs.

Bevorzugt weist das Werkzeug weiterhin eine Bohrhilfe mit mindestens einer Öffnung auf, die mit dem Haltegriff verbunden sein kann, insbesondere über mindestens eine zweite Sollbruchstelle. Durch Abtrennen der Bohrhilfe an der zweiten Sollbruchstelle kann die Bohrhilfe verwendet werden, um mindestens ein Loch in einem Bereich eines Knochens präzise zu positionieren, in dem die Klammer eingesetzt werden soll. Die Bohrhilfe ist über den Haltegriff indirekt mit der Klammer verbunden. Hierdurch ist gewährleistet, dass eine auf die Klammer abgestimmte Bohrhilfe unmittelbar zur Verfügung steht und nicht separat bereitgestellt werden muss.

Ein zweiter Aspekt der Erfindung betrifft ein chirurgisches Werkzeug mit einer chirurgischen Klammer und einer Bohrhilfe. Die chirurgische Klammer weist mindestens zwei, bevorzugt genau zwei Schenkel und eine die Schenkel verbindende Basis auf. Insbesondere kann es sich um ein chirurgisches Werkzeug gemäss dem ersten Aspekt der Erfindung handeln. Gemäss dem zweiten Aspekt der Erfindung enthält die Bohrhilfe mindestens zwei Öffnungen, deren Abstand grösser ist als der Abstand der Schenkel der Klammer. Eine derartige Bohrhilfe ermöglicht es, Löcher in zwei oder Bereichen eines Knochens derart zu positionieren, dass mit Hilfe der Klammer eine Kompression des Knochens erreicht werden kann. Werden die Schenkel in die Löcher eingesetzt, so wird der Knochen zwischen den Bereichen komprimiert.

Auch ein dritter Aspekt der Erfindung betrifft ein chirurgisches Werkzeug mit einer chirurgischen Klammer und einem Haltegriff, wobei die Klammer mindestens zwei, bevorzugt genau zwei Schenkel und eine die Schenkel verbindende Basis aufweist und der Haltegriff mit der Klammer insbesondere einstückig verbunden ist. Beispielsweise kann es sich um ein wie oben beschriebenes Werkzeug handeln. Gemäss dem dritten Aspekt der Erfindung enthält das Werkzeug weiterhin mindestens einen Bohrer, der über mindestens eine dritte Sollbruchstelle mit dem Haltegriff verbunden ist. Insbesondere kann der Bohrer an einem ersten Ende über die dritte Sollbruchstelle mit dem Haltegriff verbunden sein. Ein solches Werkzeug eröffnet die Möglichkeit, zusammen mit der Klammer unmittelbar einen auf die Klammer abgestimmten Bohrer bereitzustellen, mit dem ein Loch in einen Knochen eingebracht werden kann, in das ein Schenkel der Klammer eingesetzt werden kann.

In bevorzugten Ausführungsformen ist vorgesehen, dass der Bohrer am ersten Ende eine erste Bohrerspitze aufweist und die dritte Sollbruchstelle von der ersten Bohrerspitze beabstandet ist. Die dritte Sollbruchstelle kann bevorzugt benachbart zu einem Übergangsbereich zwischen einem Schaft des Bohrers und einem konischen Abschnitt am ersten Ende angeordnet sein. Auf diese Weise wird verhindert, dass beim Abtrennen des Bohrers an der dritten Sollbruchstelle die Bohrerspitze beschädigt wird.

Vorteilhaft ist es weiterhin, wenn der Bohrer an einem zweiten Ende über mindestens eine vierte Sollbruchstelle mit einer Bohrhilfe verbunden ist (insbesondere mit einer wie oben beschriebenen Bohrhilfe), die Teil des Werkzeugs ist, mindestens eine Öffnung aufweist und über mindestens eine zweite Sollbruchstelle mit dem Haltegriff verbunden ist. Durch die Bohrhilfe können die oben erläuterten Vorteile erreicht werden.

In vorteilhaften Ausgestaltungen ist vorgesehen, dass der Bohrer am zweiten Ende eine zweite Bohrerspitze aufweist und die vierte Sollbruchstelle von der zweiten Bohrerspitze in ähnlicher Weise wie die dritte Sollbruchstelle von der erster Bohrerspitze beabstandet ist. Dabei ist es in einer Variante denkbar, dass die erste und die zweite Bohrerspitze verschieden dimensioniert sind, um beispielsweise wahlweise Löcher mit einem von zwei verschiedenen Durchmessern zu bohren. In einer zweiten Variante können die erste und die zweite Bohrerspitze auch gleich dimensioniert sein, wodurch ein Durchmesser eindeutig vorgegeben ist.

Weiterhin ist es bevorzugt, wenn der Haltegriff mindestens eine Klammeraufnahme aufweist, in welcher die vom Haltegriff abgetrennte Klammer kraft- und/oder formschlüssig einsetzbar ist. Somit ist es nach dem Abtrennen des Haltegriffs von der Klammer möglich, die Klammer mit Hilfe des Haltegriffs zu halten. Hierfür ist also kein separates Werkzeug erforderlich.

In einer möglichen Ausführungsform ist die Klammeraufnahme als eine an einem ersten Ende des Haltegriffs gebildete Vertiefung ausgebildet. Dies stellt eine baulich einfache, aber dennoch effektive Konstruktion dar.

In einem vierten Aspekt betrifft die vorliegende Anmeldung ebenfalls ein chirurgisches Werkzeug mit einer chirurgischen Klammer, einem Haltegriff und einer Bohrhilfe, wobei es sich wiederum um ein wie oben beschriebenes Werkzeug handeln kann. Dabei weist die Klammer mindestens zwei, bevorzugt genau zwei Schenkel und eine die Schenkel verbindende Basis auf. Der Haltegriff ist mit der Klammer über mindestens eine erste Sollbruchstelle einstückig verbunden, und die Bohrhilfe verfügt über mindestens eine Öffnung, die insbesondere über mindestens eine zweite Sollbruchstelle mit dem Haltegriff verbunden ist. Gemäss dem vierten Aspekt enthält das Werkzeug und insbesondere die Bohrhilfe mindestens eine Abtrennöffnung, welche derart ausgebildet ist, dass, insbesondere nach einer Abtrennung der Bohrhilfe vom Haltegriff, die noch mit dem Haltegriff verbundene Klammer insbesondere im Bereich der ersten Sollbruchstelle derart in der Abtrennöffnung aufnehmbar ist, dass die Klammer an der ersten Sollbruchstelle vom Haltegriff abtrennbar ist. Die Abtrennöffnung kann auch in einem anderen Bereich des Werkzeugs angeordnet sein, welcher vom Haltegriff für die Klammer und der Klammer trennbar ist. Somit wird durch das Werkzeug unmittelbar die Möglichkeit bereitgestellt, die Klammer vom Haltegriff abzutrennen. Dies ist insbesondere dann vorteilhaft, wenn der zu behandelnde Knochen derart instabil ist, dass ein Abtrennen des Haltegriffs bei bereits implantierter Klammer nicht möglich ist. Die Verwendung des integrierten Abtrennöffnung reduziert auch das Risiko zerstörter Operationshandschuhe, das bei rein manuellem Biegen vorhanden sein kann. Auch hierfür bedarf es keines separaten Werkzeugs, welches ausgewählt und zusätzlich zur Verfügung gestellt werden muss.

Zweckmässig ist es weiterhin, wenn die Bohrhilfe einen Biegebereich aufweist, der zwischen einem die Öffnung für die Aufnahme eines Bohrers enthaltenden Öffnungsbereich und einem die Abtrennöffnung enthaltenen Schlitzbereich angeordnet ist und der ein Biegen der Bohrhilfe ermöglicht. Auf diese Weise kann die Bohrhilfe je nach Bedarf gebogen werden, um die Zugänglichkeit zu dem zu behandelnden Knochen zu vereinfachen oder überhaupt erst zu ermöglichen.

Ebenfalls vorteilhaft ist es, wenn die erste Sollbruchstelle gegenüber einer Begrenzungsfläche der Basis, welche dem Haltegriff zugewendet ist, versenkt ist. Diese Ausgestaltung bewirkt, dass allfällige scharfe Kanten, die beim Abtrennen des Halteteils entstehen, ebenfalls gegenüber der Begrenzungsfläche der Basis versenkt sind. Hierdurch entstehen beim Abtrennen auch an der Basis der Klammer keine scharfen Kanten, und zwar auch nicht an der von den Schenkeln abgewandten Seite der Basis. Dies trägt zur weiteren Schonung des Körpergewebes bei.

Weitere Vorteile entstehen, wenn die Bohrhilfe eine Einschlagkerbe zum Einschlagen der vom Haltegriff abgetrennten Klammer in einen Knochen aufweist, wobei die Einschlagkerbe bevorzugt an einem der Klammeraufnehme gegenüberliegenden zweiten Ende des Haltegriffs angeordnet ist.

Die Erfindung betrifft allgemeiner auch einstückige chirurgische Werkzeuge, die mindestens zwei Elemente aufweisen, die mindestens teilweise durch Sollbruchstellen und/oder Sollbiegestellen miteinander verbunden sind, wobei mindestens eines der Elemente, bevorzugt mindestens zwei der Elemente, aus der folgenden Liste ausgewählt ist/sind:
- mindestens eine chirurgische Klammer mit mindestens zwei, bevorzugt genau zwei Schenkeln und einer die Schenkel verbindenden Basis;
- mindestens einen chirurgischen Bohrer;
- mindestens ein chirurgisches Skalpell;
- mindestens eine chirurgische Tastnadel, mit dem beispielsweise die Tiefe eines in einem Knochen gebohrten Lochs messbar ist;
- mindestens eine Skala;
- mindestens eine chirurgische Schiebehilfe, insbesondere zum Einschieben einer chirurgischen Tastnadel;
- mindestens einen Bestandteil, insbesondere einen Zuschnitt für mindestens einen Arm oder einen Teil eines Zuschnitts für mindestens einen Arm, einer chirurgischen Zange;
- mindestens einen Bestandteil, insbesondere einen Zuschnitt für einen Griff oder einen Teil eines Zuschnitts für mindestens einen Griff, eines chirurgischen Schraubendrehers oder eines chirurgischen Skalpells;
- mindestens einen chirurgischen K-Draht.

Unter die zuletzt genannten Werkzeuge fallen beispielsweise auch solche Werkzeuge, welche insgesamt einen Zuschnitt für einen Arm einer chirurgischen Zange oder einen Zuschnitt für einen Griff eines chirurgischen Schraubendrehers oder eines chirurgischen Skalpells bilden, wobei der Zuschnitt mindestens zwei Elemente oder Teile enthält, die über eine Sollbiegestelle miteinander verbunden sind, an der der Zuschnitt gebogen werden kann, um einen Arm einer Zange beziehungsweise einen Griff eines Schraubendrehers oder Skalpells zu erhalten.

Weitere Aspekte der Erfindung betreffen chirurgische Werkzeuge, welche auf der Basis von Zuschnitten aus Blechen bereitgestellt werden können. Diese Werkzeuge können als individuelle Werkzeuge und bevorzugt in Kombination mit den vorstehend beschriebenen Werkzeugen und Zuschnitten eingesetzt werden.

Beispielsweise können flächige Zuschnitte verwendet werden, um Kompressionszangen für K-Drähte oder Zangen für eine Distraktion bereitzustellen.

Um solche Zangen bereitzustellen, werden zwei flächige Zuschnitte vorgeschlagen. Einer dieser Zuschnitte kann bevorzugt mit einem verpressten Stift versehen werden. Durch Biegen können die beiden Zuschnitte in eine Form gebracht werden, so dass sie zwei Arme einer Zange bilden, wenn sie zusammengesetzt sind. Entsprechend betrifft dieser Aspekt einen Satz aus zwei Zuschnitten, welche derart biegbar und in einem Schwenkpunkt miteinander schwenkbar verbindbar sind, dass die schwenkbar zusammengesetzten, gebogenen Zuschnitte zwei Arme einer Zange bilden. Das Biegen kann an einer oben genannten Sollbiegestelle vollzogen werden.

Es ist weiter denkbar, auch einen K-Draht aus einem Blech zu erzeugen, insbesondere durch dreidimensionales Laserschneiden oder zusätzliche Bearbeitungsschritte wie Fräsen oder Schleifen. Insbesondere kann ein solcher K-Draht eine optimierte Spitze aufweisen. Der K-Draht kann auch als dreikantiger K-Draht ausgebildet sein.

Eine weitere Anwendungsmöglichkeit betrifft einen Zuschnitt, der zum Herstellen eines Griffs z. B. für einen Schraubendreher oder für ein Skalpell verwendbar ist. Der Zuschnitt weist mindestens zwei, bevorzugt drei Arme zum Bilden von Seitenflächen auf. Die Seitenflächen lassen sich zu einem Griff umbiegen. Dabei sind die Seitenflächen an einem hinteren Ende mittels eines Verbindungsbereichs miteinander verbunden und an einem vorderen Ende mit Konturen versehen, die bevorzugt durch Umbiegen eine Schnittstelle zum Einsetzen von Schraubendreherklingen, insbesondere für AO-, Dental-, Stryker- oder ähnliche Ansätze oder Skalpellklingen bilden. Das Umbiegen kann an einer oben genannten Sollbiegestelle vollzogen werden.

Ebenfalls denkbar ist es, einen temporären Plattenfixierer (ein sogenannter Oliven-K-Draht) als Zuschnitt aus einem Blech bereit zu stellen.

Während die letztgenannten verschiedenen Werkzeuge auch für sich alleine vorteilhaft einsetzbar sind, versteht es sich von selbst, dass bevorzugt auch Kombinationen mit anderen Werkzeugen oder Bauteilen denkbar sind. Die Bereitstellung von Zuschnitten aus Blechen erlaubt es, flexibel je nach gewünschter Anwendung einzelne Werkzeuge oder Bauteile miteinander zu kombinieren.

Auch diese Werkzeuge weisen bevorzugt mindestens einen Haltegriff auf, z.B. zum Halten eines chirurgischen Gegenstandes, insbesondere einer chirurgischen Klammer und/oder mindestens eine chirurgische Bohrhilfe mit mindestens einer Öffnung auf.

Die Elemente des Werkzeugs sind bevorzugt aufeinander abgestimmt. Die einstückige Verbindung ermöglicht eine verlustsichere und fehlerfreie Bereitstellung mehrerer solcher aufeinander abgestimmter Elemente.

Die erfindungsgemässen chirurgischen Werkzeuge können beispielsweise aus einem Metall wie etwa Stahl oder aus Titan, Kunststoff, Nitinol oder Keramik bestehen. Sie können beispielsweise durch mechanisches Ausstanzen, Laserschneiden, Fräsen, Wasserstrahlschneiden oder Erodieren aus einem Blech oder durch mechanische Bearbeitung (beispielsweise Fräsen, Bohren oder Schleifen) und Biegen, Prägen oder Verpressen hergestellt werden. Das Blech kann eine Dicke im Bereich von 0,1 mm bis 8 mm aufweisen. Die Schenkel der Klammer haben eine Dicke von 0,1 mm bis 8 mm und können einen Abstand voneinander aufweisen, der im Bereich von 2 mm bis 40 mm, bevorzugt von 8 mm bis 10 mm liegt.

Nachfolgend wird die Erfindung anhand eines Ausführungsbeispiels im Detail erläutert. Dabei zeigt
- Figur 1:: ein erstes erfindungsgemässes chirurgisches Werkzeug in einer Draufsicht;
- Figur 2:: ein zweites erfindungsgemässes chirurgisches Werkzeug in einer perspektivischen Ansicht;
- Figur 3:: eine alternative Ausführungsform des Werkzeugs aus Figur 1;
- Figur 4:: eine vergrösserte Darstellung eines Ausschnitts A aus Figur 1;
- Figur 5:: eine vergrösserte Darstellung eines Ausschnitts B aus Figur 1;
- Figur 6a:: ein erster Teil einer Kompressionszange für K-Drähte (links abgewickelt und rechts gebogen);
- Figur 6b:: ein zweiter Teil einer Kompressionszange für K-Drähte (links abgewickelt und rechts gebogen);
- Figur 6c:: eine aus den Teilen gemäss Figur 6a und 6b zusammengebaute Kompressionszange;
- Figur 6d:: einen K-Draht;
- Figur 7:: eine alternative Ausführungsform einer Kompressionszange;
- Figur 8a:: einen Zuschnitt zur Herstellung eines Schraubendrehergriffs;
- Figur 8b:: den Zuschnitt aus Figur 8a mit abgebogenen Enden;
- Figur 8c:: den zu einem offenen Griff gebogenen Zuschnitt aus Figur 8a;
- Figur 8d:: den Griff auf Figur 8c mit eingesetzter Schraubendreherklinge;
- Figur 8e:: den Schraubendreher aus Figur 8d mit geschlossenem Griff;
- Figur 8f:: eine perspektivische Darstellung der Schnittstelle zwischen Schraubendreher und Schraubendrehergriff aus Figur 8e und
- Fig. 9: einen temporären Plattenfixierer ("Oliven-K-Draht") .

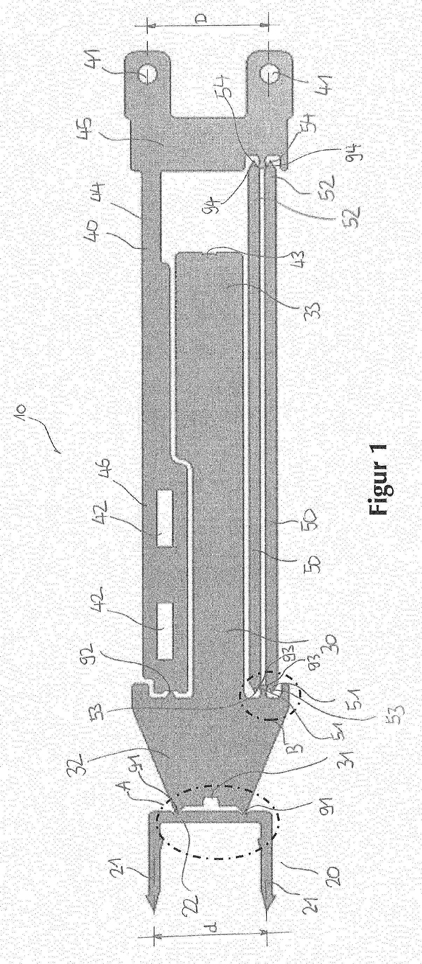

Das in Figur 1 dargestellte erste erfindungsgemässe chirurgische Werkzeug 10 enthält eine chirurgische Klammer 20 mit zwei Schenkeln 21 und einer die Schenkel verbindenden Basis 22. Ein Haltegriff 30 ist über zwei Sollbruchstellen 91 mit der Basis 22 verbunden. Die ersten Sollbruchstellen 91 sind gegenüber einer Begrenzungsfläche der Basis 22, welche dem Haltegriff 30 zugewendet ist, versenkt.

Das Werkzeug 10 weist weiterhin eine Bohrhilfe 40 mit zwei Öffnungen 41 auf. Die Bohrhilfe 40 ist über eine zweite Sollbruchstelle 92 mit dem Haltegriff 30 verbunden. Die Öffnungen 41 der Bohrhilfe weisen einen Abstand D auf, der grösser ist als der Abstand d der Schenkel 21 der Klammer 20. Überdies verfügt in diesem Ausführungsbeispiel die Bohrhilfe 40 des Werkzeugs 10 über eine Abtrennöffnung in Form von zwei Abtrennschlitzen 42. In alternativen erfindungsgemässen Ausführungsformen könnte eine Abtrennöffnung auch in einem anderen Element des einstückigen Werkzeugs 10 gebildet sein. Die Bohrhilfe 40 weist zudem einen Biegebereich 44 auf, der zwischen einem die Öffnungen 41 enthaltenden Öffnungsbereich 45 und einem die Abtrennöffnung enthaltenen Schlitzbereich 46 angeordnet ist. Die Bohrhilfe 40 weist ausserdem eine Einschlagkerbe 43 zum Einschlagen der vom Haltegriff 30 abgetrennten Klammer 20 in einen Knochen auf. Die Einschlagkerbe 43 ist an einem einer Klammeraufnahme 31 gegenüberliegenden zweiten Ende 33 des Haltegriffs 30 angeordnet. Die Funktionen des Abtrennschlitzes 42, des Biegebereichs 44 und der Einschlagkerbe 43 werden weiter unten noch im Detail erläutert.

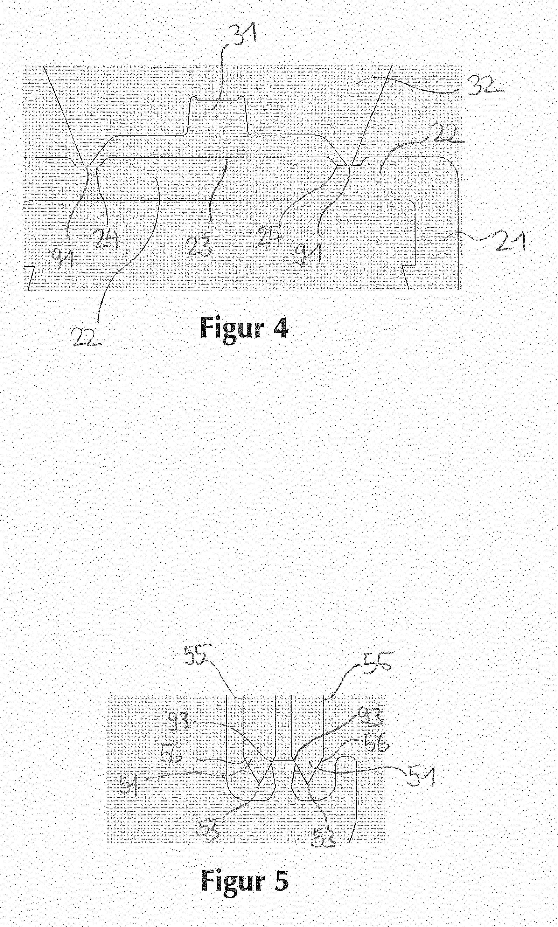

Das Werkzeug 10 enthält weiterhin zwei Bohrer 50, die an jeweiligen ersten Enden 51 über dritte Sollbruchstellen 93 mit dem Haltegriff 30 verbunden sind (siehe auch Fig. 5). Die Bohrer 50 weisen an ihren Enden 51 eine jeweilige erste Bohrerspitze 53 auf, wobei die dritten Sollbruchstellen 93 von den ersten Bohrerspitzen 53 beabstandet sind. An ihren jeweiligen zweiten Enden 52 sind die Bohrer 50 über vierte Sollbruchstellen 94 mit der Bohrhilfe 40 verbunden. Auch an den zweiten Enden 52 weisen die Bohrer 50 eine jeweilige zweite Bohrerspitze 54 auf, wobei die vierten Sollbruchstellen 94 von den zweiten Bohrerspitzen 54 beabstandet sind.

Die Klammeraufnahme 31 ist als eine an einem ersten Ende 32 des Haltegriffs 30 gebildete Vertiefung ausgebildet. Auch die Funktion der Vertiefung wird nachfolgend erläutert.

Zur Verwendung des Werkzeugs 10 kann in einem ersten Schritt die Bohrhilfe 40 an der zweiten Sollbruchstelle 92 vom Haltegriff 30 getrennt werden. Bevorzugt werden dabei auch die Bohrer 50 an den dritten Sollbruchstellen 93 vom Haltegriff 30 getrennt, während sie zunächst an den vierten Sollbruchstellen 94 mit der Bohrhilfe 40 verbunden bleiben. Alternativ können beim Abtrennen der Bohrhilfe 40 vom Haltegriff 30 die Bohrer 50 an den vierten Sollbruchstellen 94 von der Bohrhilfe 40 abgetrennt werden, während die Bohrer 50 über die dritten Sollbruchstellen 93 zunächst mit dem Haltegriff 30 verbunden bleiben.

Nachdem somit die Bohrhilfe 40 vom Haltegriff 30 getrennt wurde, können die Schenkel 21 der noch mit dem Haltegriff 30 verbundenen Klammer 20 in die Abtrennschlitze 42 eingeführt werden, sodass die ersten Sollbruchstellen 91 benachbart zu den Abtrennschlitzen 42 zu liegen kommen. Durch Biegen des Haltegriffs 30 und der damit noch verbundenen Klammer 20 kann die Klammer 20 an den ersten Sollbruchstellen 91 sauber und kontrolliert vom Haltegriff 30 abgetrennt werden. Aufgrund der Anordnung der Sollbruchstellen 91 an der Basis 22 entstehen höchstens an der den Schenkeln 21 abgewandten Seiten scharfe Bruchkanten.

Vor oder nach dem Abtrennen der Klammer 20 vom Haltegriff 30 kann die Bohrhilfe 40 in an sich bekannter Weise verwendet werden, um die Positionen zweier Löcher in zwei Bereichen eines Knochens (z. B. über einer Fraktur oder in zwei Knochen über einem zu fusionierenden Gelenk) festzulegen. Bei Bedarf kann die Bohrhilfe 40 im verjüngten Biegebereich 44 gebogen werden, um die Zugänglichkeit zu ermöglichen. Die Löcher können mit Hilfe der vom Haltegriff 30 und von der Bohrhilfe 40 abgetrennten Bohrer 50 eingebracht werden.

Die vom Haltegriff 30 abgetrennte Klammer 20 kann anschliessend mit ihrer Basis in die Vertiefung 31 des Haltegriffs 30 eingeklemmt und somit kraft- und/oder formschlüssig gehalten werden. Die Vertiefung 31 dient dabei als Abrutschsicherung beim Einklopfen. Die beiden Schenkel 21 der Klammer können sodann in die im Knochen eingebrachten Löcher eingesetzt werden. Die Einschlagkerbe 43 ermöglicht ein feines Einschlagen der Klammer 20 in den Knochen, wodurch sie noch näher an den Knochen herangebracht werden kann.

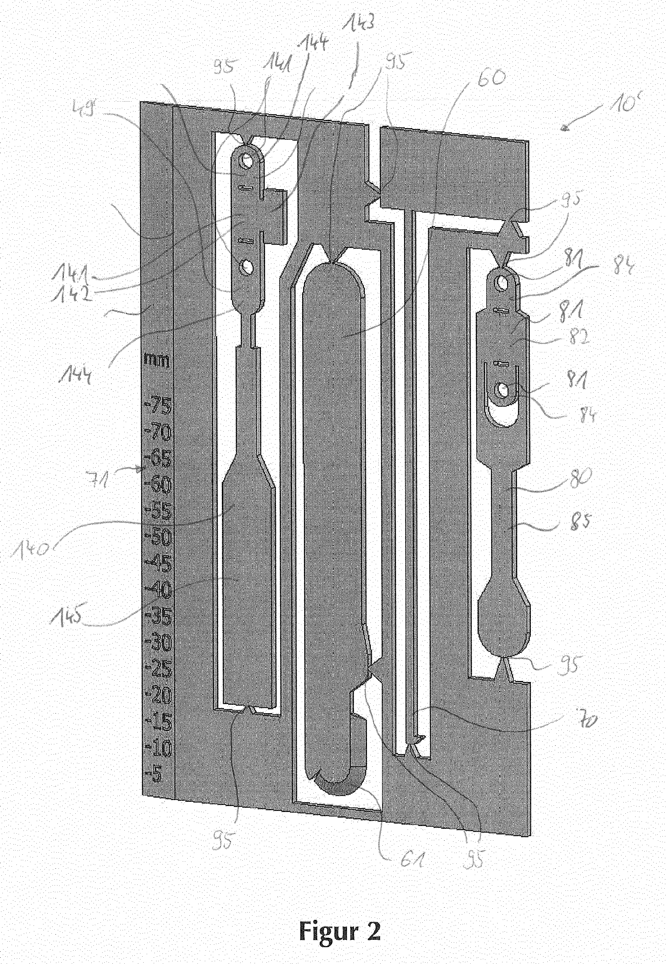

Die Figur 2 zeigt ein zweites erfindungsgemässes chirurgisches Werkzeug 10', welches ebenfalls einstückig ausgebildet ist. Es enthält die folgenden Elemente, die über Sollbruchstellen 95 miteinander verbunden sind:
- eine chirurgische Bohrhilfe 140 mit zwei Öffnungen 141;
- ein chirurgisches Skalpell 60 mit einer Klinge 61;
- eine Schiebehilfe 80 mit zwei Öffnungen 81;
- eine Skala 71;
- eine Tastnadel 70.

Die Bohrhilfe 140 verfügt über einen Haltegriff 145 und eine damit verbundene Lasche 141. Die Lasche 141 enthält einen mittleren Bereich 142, von dem seitlich ein Rechteck 143 absteht, sowie zwei Endbereiche 144, die jeweils eine der Öffnungen 141 enthalten. Zur Verwendung werden das Rechteck 143 und die Endbereiche 144 um jeweils 90° auf dieselbe Seite geklappt. Das abstehende Rechteck 143 wird als Anschlag für die Endbereiche 144 benutzt. So stehen die beiden Öffnungen 141 axial ausgerichtet einander gegenüber. Ein Bohrer kann dann axial durch beide Öffnungen 141 geschoben und auf diese Weise geführt werden.

Die Schiebehilfe 80 verfügt über einen Haltegriff 85 und eine damit verbundene Lasche 81. Die Lasche 81 enthält einen mittleren Bereich 82 sowie zwei Endbereiche 84, die jeweils eine der Öffnungen 81 enthalten. Zur Verwendung werden die Endbereiche 84 um jeweils 90° auf dieselbe Seite geklappt. So stehen die beiden Öffnungen 81 axial ausgerichtet einander gegenüber. Dann wird die Tastnadel 70 durch die ausgerichteten Öffnungen 81 geschoben. Zum Messen der Tiefe eines in einem Knochen gebohrten Lochs wird die Tastnadel 70 in das Loch hineingeschoben. Dann wird die Schiebehilfe 80 bis an den Anschlag geschoben. Das Konstrukt wird so gehalten, aus dem Loch gezogen und an eine am Werkzeug 10' angeordnete Skala 71 gehalten und abgelesen. Auf diese Weise kann die Tiefe des Lochs bestimmt werden.

Das Werkzeug 10' kann auf eine oder mehrere spezielle Indikationen ausgerichtet sein und die hierfür nötigen und geeigneten Elemente aufweisen, die durch Abtrennen an den Sollbruchstellen voneinander getrennt und verwendet werden können.

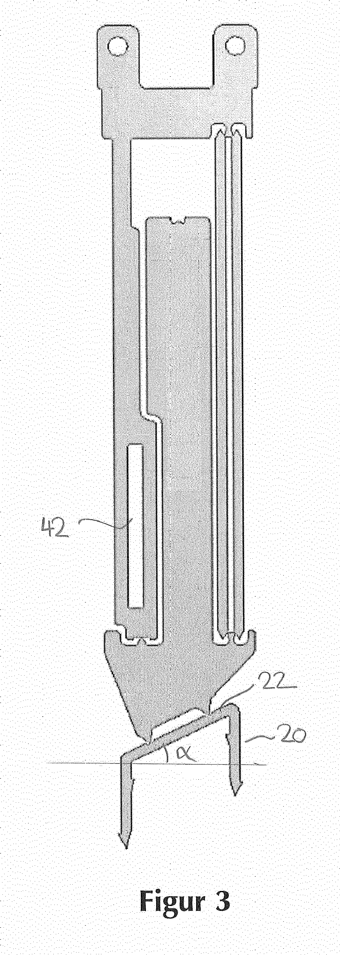

Figur 3 zeigt eine alternative Ausführungsform des Werkzeugs aus Figur 1. Nachfolgend werden die Unterschiede im Vergleich zu der Ausführungsform gemäss Figur 1 erläutert. Anstelle von zwei Abtrennschlitzen ist nur ein Abtrennschlitz 42 vorgesehen, der eine Breite aufweist, die etwa der Breite der Klammer 20 entspricht. Ausserdem hat die Klammer 20 eine abgeschrägte Basis 22, die unter einem Winkel α von ca. 25° verläuft.

Figur 4 zeigt eine vergrösserte Darstellung des Ausschnitts A aus Figur 1. Die Sollbruchstellen 91 sind in der Basis 22 an der dem Haltegriff zugewandten Seite 23 in einer Vertiefung 24 versenkt angeordnet.

Figur 5 zeigt eine vergrösserte Darstellung des Ausschnitts B aus Figur 1. Die Bohrer 50 weisen einen Schaft 55 und einen keilförmigen Abschnitt 56 am ersten Ende 51 auf. Der keilförmige Abschnitt 56 läuft vom Schaft 55 in die Bohrerspitze 53. Die Sollbruchstellen 93 sind beabstandet zur Bohrerspitze 53 angeordnet, benachbart zum Übergang zwischen dem Schaft 55 und dem konischen Abschnitt 56. Diese Anordnung der Sollbruchstellen 93 ist vorteilhaft, da eine Anordnung im Schaft 55 aufgrund von Grat eine grössere Bohrung produzieren würde und eine Anordnung an der Spitze zum Verlaufen des Bohrers 50 führen würde.

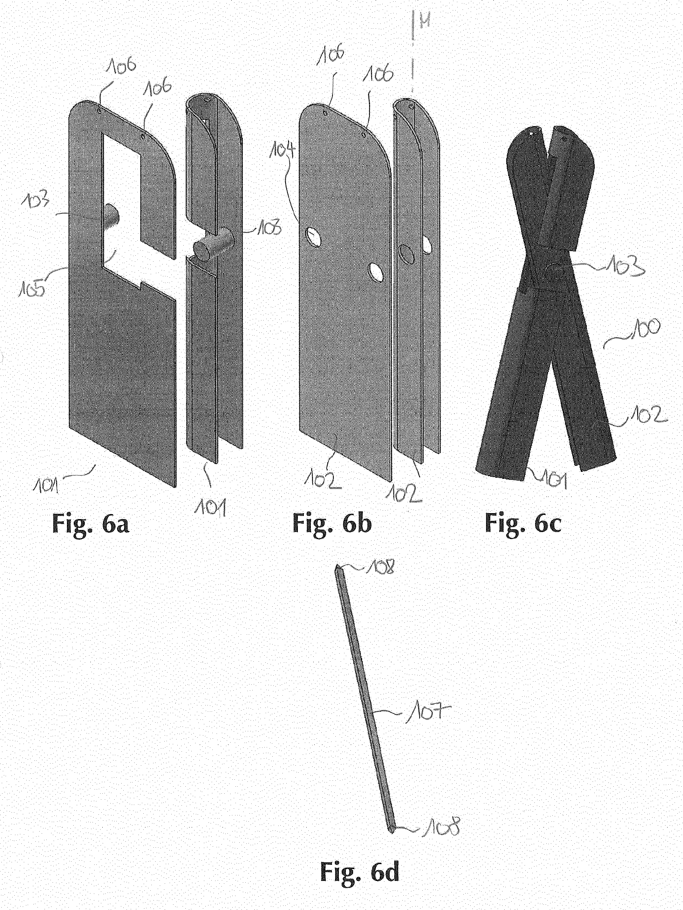

Die Figuren 6a und 6b zeigen einen ersten Arm 101 und einen zweiten Arm 102 für eine Kompressionszange 100 (siehe Figur 6c). In den Figuren 6a und 6b ist jeweils auf der linken Seite ein flächiger Zuschnitt und auf der rechten Seite eine gebogene Form des Zuschnitts gezeigt.

Der erste Arm 101 weist einen mit dem Zuschnitt 101 verpressten Stift 103 auf. Im Zuschnitt 101 ist ausserdem eine Aussparung 105 zur Aufnahme des gebogenen zweiten Arms 102 (siehe Figur 6c) vorgesehen. Ausserdem sind Öffnungen 106 zum Aufnehmen eines K-Drahtes vorgesehen. Der Zuschnitt für den ersten Arm 101 wird entlang einer Mittelachse M gebogen, sodass sich ein U-förmiger Querschnitt ergibt. Der Zuschnitt kann flach oder in vorgebogener Form bereitgestellt werden. Ebenso kann der Stift 103 vorverpresst, separat oder als abtrennbares Teil am Werkzeug 10 bereitgestellt werden.

Figur 6b zeigt einen Zuschnitt für den zweiten Arm 102. Der zweite Arm 102 ist mit zwei Öffnungen 104 versehen, durch welche der Stift 103 führbar ist. Ebenfalls sind im zweiten Arm Öffnungen 106 vorgesehen. Der zweite Arm wird (siehe Figur 6b, rechts) ebenfalls entlang einer Mittelachse M gebogen, so dass ein U-förmiger Querschnitt entsteht. Durch Zusammensetzen des ersten Arms 101 und des zweiten Arms 102 wird die in Figur 6c gezeigte Kompressionszange 100 gebildet. Dazu wird der zweite Arm 102 durch die Aussparung 105 eingeführt und auf den Stift 103 aufgesetzt.

Figur 6d zeigt einen K-Draht 107. Der K-Draht 107 ist durch 3D-Laserschneiden aus einem Blech ausgeschnitten oder durch zusätzliches Fräsen, Schleifen oder Pressen eines lasergeschnittenen Teils hergestellt. Gleichzeitig können dreidimensionale Spitzen 108 geformt werden.

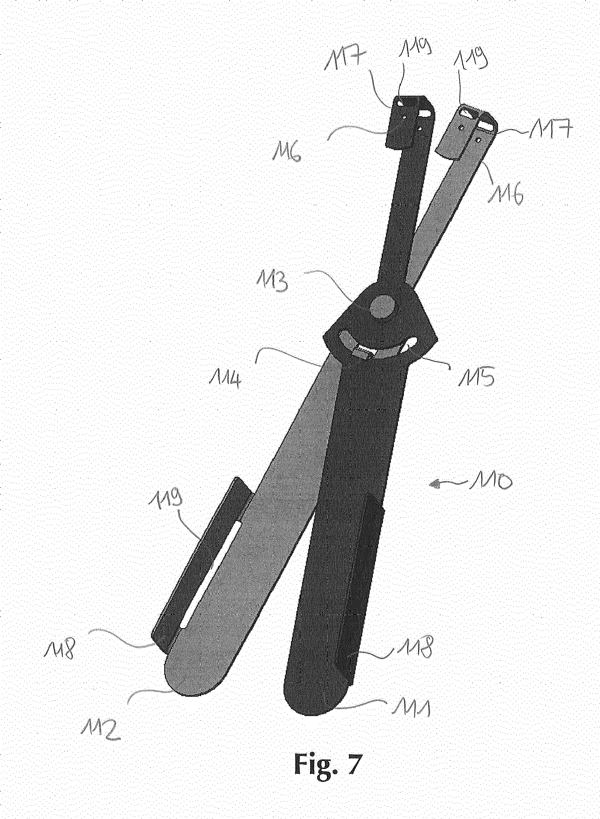

Figur 7 zeigt eine alternative Ausführungsform einer Kompressionszange 110, welche aus einem ersten Arm 111 und einem zweiten Arm 112 aufgebaut ist. Der erste Arm 111 und der zweite Arm 112 sind über einen Stift 113, der mit dem ersten Arm 112 verpresst oder ausgebogen und eingerenkt ist, schwenkbar miteinander verbunden. Der erste Arm 111 und der zweite Arm 112 sind beide als flächige Zuschnitte aus einem Blech hergestellt. Der zweite Arm 111 weist eine Führungsbahn 115 auf, entlang welcher ein Anschlag 114 des zweiten Arms 112 führbar ist. Der erste Arm 111 und der zweite Arm 112 weisen abgebogene Abschnitte 118 auf, welche einen Handgriff bilden. An einem dem Handgriff 118 abgewandten Ende ist ein Kompressionskopf 117 gebildet. Der Kompressionskopf 117 weist Öffnungen 116 sowie schlitzförmige Öffnungen 119 auf. Die schlitzförmigen Öffnungen 119 dienen (ähnlich wie schlitzförmige Öffnungen 119 im Bereich des Handgriffs 118) der einfacheren Biegbarkeit.

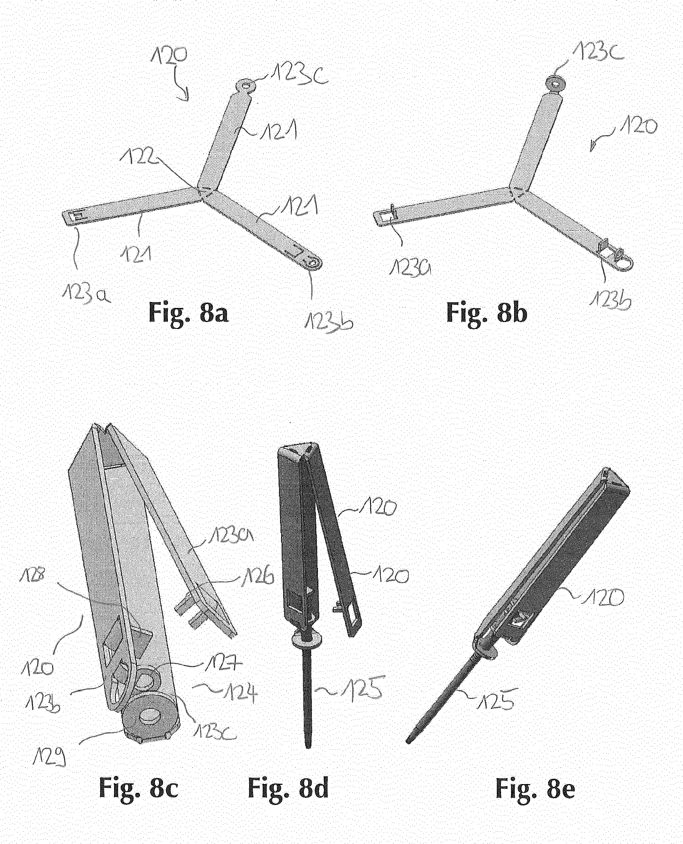

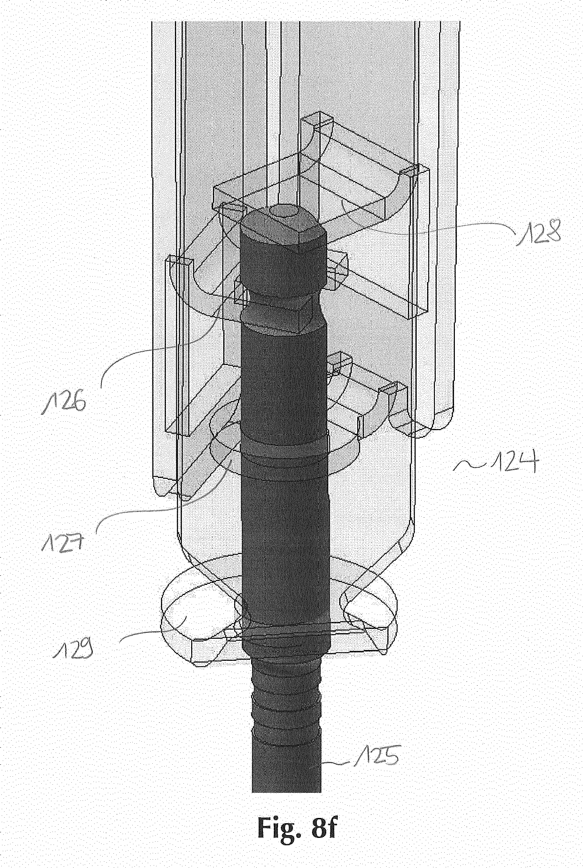

Figuren 8a bis 8f zeigen verschiedene Konfigurationen eines Griffs 120 für einen Schraubendreher. Der Griff 120 ist aus einem flächigen Zuschnitt gebildet. In Figur 8a ist der flächige Zuschnitt für den Griff 120 gezeigt. Der Zuschnitt weist einen Verbindungsbereich 122 auf, von dem sich drei Arme 121 sternförmig erstrecken. Schlitze zwischen dem Verbindungsbereich 122 und den Armen 121 vereinfachen die Biegbarkeit. An den Enden der Arme 121 sind Endbereiche 123a, 123b und 123c angeordnet, welche im zusammengesetzten Griff 120 eine Schnittstelle zur Aufnahme eine Klinge 125 (siehe Figur 8f) bilden.

Figur 8b zeigt den Zuschnitt aus Figur 8a mit einzelnen, abgebogenen Elementen im Bereich der Endbereiche 123a, 123b und 123c.

Zum Bilden des Griffs 120 werden die Arme 121 um 90° gebogen, sodass sich ein dreidimensionaler Körper ausbildet (siehe Figur 8c). Durch die abgebogenen Teile in den Endbereichen 123a, 123b und 123c bildet sich eine Schnittstelle 124 für die Klinge 125 aus. Eine Führungsöffnung 129 dient zur Aufnahme des Schafts der Klinge 125. Eine Drehsicherungsöffnung 127 dient zur Aufnahme eines unrunden Teils der Klinge 125 zum Übertragen eines Drehmoments. Ein Endanschlag 128 definiert eine axiale Endposition für die Klinge 125. Zwei nach innen gerichtete Vorsprünge bilden eine axiale Sicherung 126, in welche die Klinge 125 mit einer Einschnürung formschlüssig eingreifen kann.

Figur 8d zeigt den Griff 120 mit eingesetzter Klinge 125. Nach Einsetzen der Klinge wird der letzte Arm (hier der Arm mit dem Endstück 123a) in eine Endposition gebogen, so dass die axiale Sicherung 126 in die Aussparung an der Klinge 125 eingreift (siehe Figur 8e).

Figur 8f zeigt eine vergrösserte Darstellung der Schnittstelle 124 zur Aufnahme der Klinge 125.

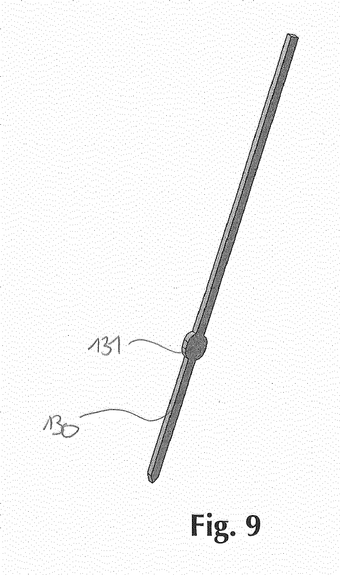

In Figur 9 ist ein temporärer Plattenfixierer ("Oliven-K-Draht") 130 gezeigt. Der temporäre Plattenfixierer 130 hat die Form eines K-Drahts mit einer Olive 131.

## Patentansprüche

1. Chirurgisches Werkzeug (10), enthaltend
- eine chirurgische Klammer (20) mit mindestens zwei, bevorzugt genau zwei Schenkeln (21) und einer die Schenkel (21) verbindenden Basis (22),
- einen mit der Klammer (20) einstückig verbundenen Haltegriff (30),
**dadurch gekennzeichnet, dass**
der Haltegriff (30) über mindestens eine erste Sollbruchstelle (91) mit der Basis (22) der Klammer (20) verbunden ist.

2. Chirurgisches Werkzeug (10) gemäss Anspruch 1,
wobei das Werkzeug (10) weiterhin eine Bohrhilfe (40) mit mindestens einer Öffnung (41) aufweist, wobei die Bohrhilfe (40) mit dem Haltegriff (30) verbunden ist, insbesondere über mindestens eine zweite Sollbruchstelle (92).

3. Chirurgisches Werkzeug (10), enthaltend
- eine chirurgische Klammer (20) mit mindestens zwei, bevorzugt genau zwei Schenkeln (21) und einer die Schenkel (21) verbindenden Basis (22),
- eine Bohrhilfe (40),
insbesondere chirurgisches Werkzeug (10) gemäss Anspruch 2,
**dadurch gekennzeichnet, dass**
die Bohrhilfe (40) mindestens zwei Öffnungen (41) enthält, deren Abstand (D) grösser ist als der Abstand (d) der Schenkel (21) der Klammer (20).

4. Chirurgisches Werkzeug (10), enthaltend
- eine chirurgische Klammer (20) mit mindestens zwei, bevorzugt genau zwei Schenkeln (21) und einer die Schenkel (21) verbindenden Basis (22),
- einen mit der Klammer (20) insbesondere einstückig verbundenen Haltegriff (30),
insbesondere chirurgisches Werkzeug (10) gemäss einem der vorangehenden Ansprüche,
**dadurch gekennzeichnet, dass**
das Werkzeug (10) weiterhin mindestens einen Bohrer (50) enthält, der, insbesondere an einem ersten Ende (51), über mindestens eine dritte Sollbruchstelle (93) mit dem Haltegriff (30) verbunden ist.

5. Chirurgisches Werkzeug (10) gemäss Anspruch 4,
wobei der Bohrer am ersten Ende (51) eine erste Bohrerspitze (53) aufweist und die dritte Sollbruchstelle (93) von der ersten Bohrerspitze (53) beabstandet ist.

6. Chirurgisches Werkzeug (10) gemäss einem der Ansprüche 4 und 5,
wobei der Bohrer (50) an einem zweiten Ende (52) über mindestens eine vierte Sollbruchstelle (94) mit einer Bohrhilfe (40) verbunden ist, insbesondere mit einer Bohrhilfe (40), die mindestens eine Öffnung (41) aufweist und über mindestens eine zweite Sollbruchstelle (92) mit einem Haltegriff (30) des Werkzeugs (10) verbunden ist.

7. Chirurgisches Werkzeug (10) gemäss Anspruch 6,
wobei der Bohrer (50) am zweiten Ende (52) eine zweite Bohrerspitze (54) aufweist und die vierte Sollbruchstelle (94) von der zweiten Bohrerspitze (54) beabstandet ist.

8. Chirurgisches Werkzeug (10) gemäss einem der vorangehenden Ansprüche,
wobei der Haltegriff (30) mindestens eine Klammeraufnahme (31) aufweist, in welcher die vom Haltegriff (30) abgetrennte Klammer (20) kraft- und/oder formschlüssig einsetzbar ist.

9. Chirurgisches Werkzeug (10) gemäss Anspruch 8,
wobei die Klammeraufnahme (31) als eine an einem ersten Ende (32) des Haltegriffs (30) gebildete Vertiefung (31) ausgebildet ist.

10. Chirurgisches Werkzeug (10), enthaltend
- eine chirurgische Klammer (20) mit mindestens zwei, bevorzugt genau zwei Schenkeln (21) und einer die Schenkel (21) verbindenden Basis (22),
- einen mit der Klammer (20) über mindestens eine erste Sollbruchstelle (91) einstückig verbundenen Haltegriff (30),
- eine Bohrhilfe (40) mit mindestens einer Öffnung (41), die insbesondere über mindestens eine zweite Sollbruchstelle (92) mit dem Haltegriff (30) verbunden ist,
insbesondere chirurgisches Werkzeug (10) gemäss einem der vorangehenden Ansprüche,
**dadurch gekennzeichnet, dass** das Werkzeug und insbesondere die Bohrhilfe (40) mindestens eine Abtrennöffnung (42) enthält, welche derart ausgebildet ist, dass, insbesondere nach einer Abtrennung der Bohrhilfe (40) vom Haltegriff (30), die Klammer insbesondere im Bereich der ersten Sollbruchstelle (91) derart in der Abtrennöffnung (42) aufnehmbar ist, dass die Klammer (20) an der ersten Sollbruchstelle (91) vom Haltegriff (30) abtrennbar ist.

11. Chirurgisches Werkzeug (10) gemäss Anspruch 10,
wobei die Bohrhilfe (40) einen Biegebereich (44) aufweist, der zwischen einem die Öffnung (41) enthaltenden Öffnungsbereich (45) und einem die Abtrennöffnung (42) enthaltenden Schlitzbereich (46) angeordnet ist und der ein Biegen der Bohrhilfe (40) ermöglicht.

12. Chirurgisches Werkzeug (10) gemäss einem der vorangehenden Ansprüche,
wobei die erste Sollbruchstelle (91) gegenüber einer Begrenzungsfläche (55) der Basis (22), welche dem Haltegriff (30) zugewendet ist, versenkt ist.

13. Chirurgisches Werkzeug (10) gemäss einem der Ansprüche 2 bis 12,
wobei die Bohrhilfe (40) eine Einschlagkerbe (43) zum Einschlagen der vom Haltegriff (30) abgetrennten Klammer (20) in einen Knochen aufweist, wobei die Einschlagkerbe (43) bevorzugt an einem der Klammeraufnahme (31) gegenüberliegenden zweiten Ende (33) des Haltegriffs (30) angeordnet ist.

14. Einstückiges chirurgisches Werkzeug (10; 10'), insbesondere gemäss einem der vorangehenden Ansprüche,
wobei das Werkzeug (10; 10') mindestens zwei der folgenden Elemente (20, 30, 40, 50, 60, 70) aufweist, die durch Sollbruchstellen (91, 92, 93, 94) miteinander verbunden sind:
- mindestens einen Haltegriff (30) zum Halten eines chirurgischen Gegenstandes (20), insbesondere einer chirurgischen Klammer (20);
- mindestens einen chirurgischen Bohrer (50);
- mindestens ein chirurgisches Skalpell (60);
- mindestens eine chirurgische Tastnadel (70);
- mindestens eine Skala (71);
- mindestens eine chirurgische Schiebehilfe (80), insbesondere zum Einschieben einer chirurgischen Tastnadel (70);
- mindestens einen Bestandteil, insbesondere einen Zuschnitt für mindestens einen Arm (101; 102) oder einen Teil eines Zuschnitts für mindestens einen Arm (101; 102), einer chirurgischen Zange (100; 110);
- mindestens einen Bestandteil, insbesondere einen Zuschnitt für einen Griff (120) oder einen Teil eines Zuschnitts für mindestens einen Griff (120), eines chirurgischen Schraubendrehers oder eines chirurgischen Skalpells;
- mindestens einen chirurgischen K-Draht (130).

15. Chirurgisches Werkzeug (10) gemäss Anspruch 14, wobei das Werkzeug zusätzlich aufweist:
- mindestens eine chirurgische Klammer (20) mit mindestens zwei, bevorzugt genau zwei Schenkeln (21) und einer die Schenkel (21) verbindenden Basis (22) und/oder
- mindestens eine chirurgische Bohrhilfe (40; 40'; 40'') mit mindestens einer Öffnung (40; 41', 41'').
